# EUROPEAN PATENT APPLICATION

(11) **EP 4 258 279 A1**
(43) Date of publication of application: **11.10.2023**
(21) Application number: 22166948.4
(22) Date of filing: 06.04.2022
(51) Int. Cl.: G16H 40/20, G16H 50/50, A61C 9/00, G16H 70/20, A61C 7/00

(54) **DIGITAL DENTAL IMPRESSIONS**

(71) Applicant: DENTSPLY SIRONA Inc., York, PA 17401 (US); Sirona Dental Systems GmbH, 64625 Bensheim (DE)
(72) Inventor: Derzapf, Evgenij, 64625 Bensheim (DE); Koza, Andre, 64625 Bensheim (DE); Bielser, Daniel, 8050 Zürich (CH); Tanaka, Simon, 8050 Zürich (CH); Muff, Samuel, 8050 Zürich (CH); Löschhorn, Sandro, 8050 Zürich (CH)
(74) Representative: Özer, Alpdeniz

(57) **Abstract**

The present teachings relate to a method for improving digital dentistry comprising: providing a 3D digital impression of a craniofacial anatomy; the digital impression comprising a plurality of cells, providing a database of a plurality of dental procedures and their corresponding cell criteria; analyzing the cells with respect to the cell criteria; determining, in response to the analysis, which of the dental procedures the digital impression is usable for and/or which of the dental procedures the digital impression is not usable for. The present teachings also relate to a system, data, software product and a storage medium.

## Description

### TECHNICAL FIELD

The present teachings relate generally to computer-implemented methods and systems for processing digital dental impressions or models.

### BACKGROUND

A digital dental impression is a digital representation of a patient's oral anatomy. The dental impression is usable for providing for the patient dental treatments or dental objects such as surgical templates, custom devices, prosthetic restorations, or orthodontic aligners. The digital dental impression or digital impression may be captured via a dental scanner, for example an intraoral scanner. Building a good dental digital impression can take time and high-quality equipment. Sometimes, imperfections such as gaps can exist in the digital impression, which may be required to be remedied, for example, via a repeat scan. This can increase the chair time for the patient.

The applicant has realized that there is a need for improved methods and systems which can improve acquisition and/or use of digital impressions.

### SUMMARY

At least some of the limitations associated with the foregoing can be overcome by the subject matter of the accompanying independent claims. At least some of the additional advantageous alternatives will be outlined in the dependent claims.

When viewed from a first perspective, there can be provided a computer-implemented method for improving digital dentistry, which method comprises:
- providing a three-dimensional digital impression of a craniofacial anatomy e.g., oral anatomy; wherein the digital impression comprises a plurality of cells,
- providing a database of a plurality of dental procedures and their corresponding cell criteria;
- analyzing the cells with respect to the cell criteria;
- determining, in response to the analysis, which of the dental procedures the digital impression is usable for and/or which of the dental procedures the digital impression is not usable for.

The applicant has realized by inventive effort that by doing so not only can the applicability of a given digital impression be evaluated essentially simultaneously for different dental procedures, but it can also be applied in an on-the-fly manner while acquiring the digital impression from a patient to make the acquisition of the digital impression more efficient.

For example, the analysis of the digital impression may lead to a determination that the digital impression is suitable for producing a dental crown, but not suitable for producing a dental bridge. Similar evaluations can also be done, e.g., for aligners, veneers or any other dental objects, by analyzing the cells of the dental procedure with respect to the specific cell criteria of that object.

An advantage of doing so can be that usability of the digital impression is improved. For example, even if a given digital impression is not good enough for a specific dental object, it may be good enough for another dental object or dental procedure. In such cases, the planning or dental procedure may be initiated before capturing a new digital impression. Alternatively, or additionally, the digital impression, which was captured in the past, e.g., months or longer ago, may be analyzed to check if it is still usable for a more recent dental procedure which the patient may require. Thus, unnecessary acquisition of a new digital impression may be prevented. Digital dentistry can thus be improved.

Those skilled in the art shall appreciate that the cells may be in any suitable form usable for digital impressions in the dental domain, for example, in the form of mesh, point cloud, voxels, tetrahedrons, or a combination of any two or more of these forms.

According to an aspect, the method may also comprise:
- detect, in response to the analysis, one or more deficiencies in the cells for usability in one or more of the dental procedures.

Thus, when the cells are analyzed with respect to one or more cell criteria, it may be detected if any cells have one or more deficiencies which can prevent them from being usable for one or more of the dental procedures. As non-limiting examples, the deficiencies may be any one or more of, lacking precision - global and/or local, lacking completeness, inconsistency, artifacts, mesh defects such as holes, bad triangles, improper structure such as foreign objects, other data such as color or shade. As a few non-limiting examples, the analysis may involve determining that cell precision is good locally or in parts of the digital impression, and/or globally, or for the overall digital impression. Alternatively, or additionally, the analysis may involve checking completeness, in the sense that the digital impression comprises all relevant parts or cells necessary for a given dental procedure. For example, some dental procedures may be possible but the others, not e.g., no aligner possible if the digital impression relates only a partial jaw, or precision of a full jaw digital impression is bad. Alternatively, or additionally, the analysis may involve verifying that the digital impression is consistent with respect to other patient data. For example, if the digital impression is mistakenly included in the wrong patient folder which comprises other patient data such as past digital impression and/or CT scan data and/or X-ray data and/or optical scan data and/or patient photos and/or videos, the analysis may determine the digital impression being unusable, preferably for all of the dental procedures. It can thus be prevented that wrong digital impression being used for the dental procedure. Wrong treatment and/or production of a wrong dental object can thus be prevented. Alternatively, or additionally, the analysis may involve detecting presence of any artifact which may prevent the digital impression being usable for any dental procedure. Alternatively, or additionally, the analysis may involve detecting defects such as breaks or data gaps, e.g., broken mesh, holes and/or bad triangles, presence of foreign objects in the impression which may prevent the digital impression being usable for any dental procedure. Those skilled in the art shall appreciate that it may not only be determined which category of dental procedures the digital impression is suitable for or not, but also suitability for a specific kind of dental procedure. As a non-limiting example, it may not only be determined if the digital impression is suitable for a dental crown in general, but it may also be determined which kinds of dental crowns are realizable with the digital impression and which kinds not.

Thus, it shall be appreciated that the database may also comprise, or the computing unit accessing it may also have access to data such as one or more surface scans, volumetric data, articulator data, patient photos and/or videos.

According to an aspect, the method may also comprise:
- determining, in response to the analysis, at least one location or part of the digital impression in which of the cells should be improved.

Thus, it may be identified in response to the analysis, in which part the digital impression should be improved, e.g., to remove the one or more deficiencies. Hence, it is not only provided a determination that the digital impression is not suitable for which of the dental procedures, but also how the digital impression is to be improved to remedy the deficiency. Thus, rather than repeating the whole scan to acquire a new digital impression, the existing digital impression can be repaired. This can save time and effort.

It shall be appreciated that in any case, the teachings can provide a significant advantage when applied in an on-the-fly manner, i.e., the analysis is performed while the digital impression is being acquired from a patient. For example, while the patient is in chair for getting the digital impression captured, the analysis may run continuously or intermittently to determine suitability of the digital impression for at least one dental procedure. Thus, as soon as the digital impression is in a state that it meets the respective cell criteria, the suitability for those respective dental procedures is determined. This and/or any one or more other responses can be provided at a human machine interface ("HMI") unit. Thus, the method may comprise:
- providing, at a human machine interface, one or more responses to the analysis.

Thereby, the user is informed that suitability of the digital impression for a given dental procedure has been reached. Consequently, the user is enabled to terminate the acquisition. This can reduce the acquisition time to what is necessary for obtaining a digital impression for a given purpose rather than over engineering or over working the acquisition process.

The response to the HMI may be in the form of a termination signal, e.g., for informing the user that the acquisition can be terminated. Alternatively, or additionally, the termination signal may automatically terminate the acquisition process. Hence, according to an aspect the method may comprise:
- generating a termination signal for terminating the acquisition of the digital impression in response to the analysis.

Alternatively, or additionally, the method may comprise:
- providing an initiation signal for initiating a dental procedure and/or a cell corrective procedure in response to the analysis.

Thus, the initiation signal may be used to inform the user that a given dental procedure and/or cell corrective procedure for that dental procedure can be initiated, and/or any one or both of these procedures may be initiated automatically.

The method may also comprise:
- determining, in response to the initiation signal, at least remedial action by which the cells should be improved, preferably for a pre-determined dental procedure.

Hence, it may also be determined which remedial actions need to be performed for making the digital impression suitable for the pre-determined dental procedure. An output of the determination may be provided at the HMI to assist the user in improving the digital impression and/or the remedial action may be started or performed at least partially automatically.

Alternatively, or additionally, the method may comprise:
- determining, in response to the analysis, at least one quality score for the cells.

Thus, it may be calculated at least one quality score for at least one dental procedure, and preferably for all of the dental procedures. A quality score may be indicative of the degree of suitability for the digital impression for a given dental procedure. At least one, preferably a plurality of quality scores is displayed at the HMI. The quality scores may relate to different dental procedures.

Alternatively, or additionally, according to an aspect, the cell analysis is performed via a data-driven cell analyzer logic. The data-driven analyzer logic refers to a data-driven logic trained with cell analysis training data comprising a plurality of historical digital impressions and their corresponding dental procedure data.

Those skilled in the art shall appreciate that a digital impression which comprises the corrected cells as herein generated is an optimized digital impression. Thus, when viewed from another perspective there can also be provided a data storage medium storing a digital impression comprising the corrected cells as generated in any of the herein disclosed methods. Also, there can be provided a use of a digital impression comprising the corrected cells as generated in any of the herein disclosed methods for performing a dental procedure and/or for manufacturing a dental object.

For example, there can be provided a method for performing a dental procedure and/or a method for manufacturing a dental object, comprising:
- providing a digital impression comprising the corrected cells as generated in any of the herein disclosed methods;
- performing, using the digital impression, the dental procedure and/or manufacturing of the dental object.

When viewed from another perspective, there can also be provided a system comprising means for performing the steps of any of the methods herein disclosed.

For example, there can be provided a system for digital dentistry, which system comprises one or more computing units, wherein any of the computing units is/are configured to:
- provide or access, a three-dimensional digital impression of a craniofacial anatomy; wherein the digital impression comprises a plurality of cells,
- provide or access a database of a plurality of dental procedures and their corresponding cell criteria;
- analyze the cells with respect to the cell criteria;
- determine, in response to the analysis, which of the dental procedures the digital impression is usable for and/or which of the dental procedures the digital impression is not usable for.

When viewed from another perspective, there can also be provided a computer software product, or a non-transitory computer-readable storage medium storing the product, comprising instructions which when executed by a suitable one or more computing units cause any of the computing units to perform the steps of any of the methods herein disclosed.

For example, there can be provided a computer software product, or a non-transitory computer-readable storage medium storing the product, comprising instructions which when executed by a suitable one or more computing units cause any of the computing units to:
- provide or access, a three-dimensional digital impression of a craniofacial anatomy; wherein the digital impression comprises a plurality of cells,
- provide or access a database of a plurality of dental procedures and their corresponding cell criteria;
- analyze the cells with respect to the cell criteria;
- determine, in response to the analysis, which of the dental procedures the digital impression is usable for and/or which of the dental procedures the digital impression is not usable for.

"Digital impression" refers to a digital representation or model of the patient's craniofacial anatomy, more specifically oral anatomy. The digital model includes representation of anatomical features such as the patient's teeth and/or hard and/or soft tissues in and around the patient's gums. The digital impression may be usable for diagnosis and/or providing for the patient one or more treatments and/or procedures, e.g., any one or more of: dental treatment or procedure, providing or producing dental objects such as surgical templates, custom devices, prosthetic restorations, and orthodontic aligners.

The digital impression may comprise, or it may be in the form of, surface data and/or volume data of the patient's craniofacial anatomy. Accordingly, the digital impression may be in the form of a surface model and/or a volume model. The digital impression is obtained via one or more medical scans performed on the patient.

The digital impression comprises a plurality of cells or components, which may be data points arranged in surface and/or volume distribution. For example, the digital impression may be in the form of a point cloud. Other non-limiting representations or forms of the digital impression include, two-dimensional ("2D") and/or three-dimensional ("3D") mesh, voxels, tetrahedrons, or even their suitable combination. Hence, the digital impression may comprise any one or more of: datapoint e.g., in the form of point cloud, mesh, voxel, and tetrahedron.

The digital impression may even comprise any one or more of, articulator data, patient photos or videos.

The digital impression may be provided via any medical scan such as but not limited to, optical scan, X-ray, CT scan, or any of their combinations. The digital impression may relate to the whole craniofacial anatomy, or parts of it. As a non-limiting example, the digital impression may be in the form of an intraoral surface scan performed via an intraoral scanner.

"Medical scan" may refer to any kind of scan performed on a patient, intraorally and/or extraorally. As a few non-limiting examples, the medical scan may result from a diagnosis or scanning procedure, such as any one or more of, an X-ray computed tomography ("CT"), magnetic resonance imaging ("MRI"), optical dental scan, Single Photon Emission Computed Tomography ("SPECT"), positron emission tomography ("PET"), Heidelberg retina tomography ("HRT"), optical coherence tomography ("OCT"), ultrasound tomography, cone beam computer tomography ("CBCT"), for example dental CBCT and/or dental MRI or any combinations or variations of tomography imaging techniques as e.g. PET-CT or angiography.

"Anatomical feature" refers to information related to a specific craniofacial anatomy, such as oral anatomy of the patient. For example, an anatomical feature may be information related to a specific tooth or a group of teeth. Thus, anatomical features may even refer to information related to any one or more intraoral structures such as, dentition, gingiva, nerve channel, extraction site, jawbone, and condyle. Alternatively, or in addition, intraoral structures may be one or more artificial structures such as one or more dental replacements, e.g., dental crown, braces, veneer, bridge. Alternatively, or in addition, an intraoral structure may in some cases even be a scan body (or scanbody) or other natural and/or artificial structure attached to the jaw of the patient. Alternatively, or additionally, the anatomical feature may even be information related to a pathology or condition. As non-limiting examples, pathology or condition may be any one or more of, fracture of tooth or bone, caries, radiolucency, impaction, or any other characterizable state of the oral anatomy or any part thereof.

The anatomical feature may for example be a shape of a specific tooth and/or a location or position of the tooth in an absolute manner or relative to other intraoral structures. Alternatively, or additionally, the anatomical feature may be color and/or texture of an anatomical part or intraoral structure.

"Cell criteria" refers to one or more criterion which are indicative of suitability or useability of the digital impression for at least one dental procedure. Preferably, each different dental procedure is associated with their corresponding cell criteria. Dependent upon the type of digital impression, the criteria may include consistency criteria which is indicative of general validity of the cells, e.g. a mesh. In such a case, the cell criteria may include measures such as adjacency correctness and vertex triangle relationship correctness. This cell criteria may depend on the specification of the cells or mesh.

It shall be appreciated that the cell criteria may apply locally and/or globally to the digital impression. By local cell criteria, it is meant those criteria which apply to parts of a specific digital impression. By global cell criteria, it is meant those criteria which apply to a specific digital impression as a whole. Thus, in some cases, the cell criteria may even be overall surface and/or volume criteria related to their respective digital impression.

For ease of understanding, and without limitation of the generality of the present teachings, in the following the various cell criteria shall be explained with reference to a mesh. Those skilled in the art shall appreciate that from this, similar criteria can be defined also of other kinds of cells. It shall also be appreciated that mesh topology is usually based on, but not limited to, triangles. Thus, the following criteria also apply, analogously, to quadrilateral- or other simple polygon-(n-gon)-based topologies.

Alternatively, or additionally, the cell criteria may include watertightness criteria which is indicative for example of if the mesh surface divides space into two parts (e.g., inner vs. outer space). For example, the watertightness criteria may evaluate that no holes are existing in the mesh surface.

Alternatively, or additionally, the cell criteria may include flipped triangles criteria, which evaluates folded regions on the mesh surface, where the surface normals of two neighboring triangles are approximately in opposite direction (e.g., subject to a threshold value).

Alternatively, or additionally, the cell criteria may include flipped normals criteria, which evaluates whether the surface normals point in the correct direction, e.g. to the outside subspace in case of a watertight surface mesh.

Alternatively, or additionally, the cell criteria may include normal sets criteria, which evaluates regions on the surface mesh which are identified to have same or similar normals, e.g., flat regions.

Alternatively, or additionally, the cell criteria may include triangle size criteria, which evaluates size for triangles of associated elements, such as one or more of: edge length, edge length ratios, angles between edges, and triangle area.

Alternatively, or additionally, the cell criteria may include component number criteria, which evaluates number of independent mesh components. In case of watertight, non-intersecting meshes, components may represent independent objects. This analysis may be suitable for fragment or artifact detection.

Alternatively, or additionally, the cell criteria may include self-intersection criteria, which evaluates whether the surface mesh intersects with itself.

In response to analyzing the cells with respect to the cell criteria, one or more cell corrective process or repair attempts can be executed. The cell corrective process may have different levels of aggressivity. Mild or minor modifications may include mesh operations which do not alter the geometry (e.g., triangle subdivision), but more intensive mesh alterations may also be performed. The cell corrective process or procedure may even be performed in a closed loop manner, such that it is evaluated continuously or intermittently in the process: input mesh quality, mesh quality after successful or partially successful mesh repair, or all of them.

"Dental procedure" refers to any procedure which involves use of, or a procedure which can benefit from, the digital impression. As a few non-limiting examples, the dental procedure may be implant planning and/or design and/or manufacture of a dental object such as a prosthetic tooth.

"Data-driven logic" refers to a logic, which derives its functionality at least partially from training data. In contrast to a rigorous or analytical logic which is based on programmed instructions for performing a particular logical task, a data-driven logic can allow forming such instructions at least partially automatically using the training data. The use of data-driven logic can allow to describe logical and/or mathematical relationships without solving equations. This can reduce computational power and/or improve speed. Moreover, data-driven logic may be able to detect patterns or indications (e.g., in input or input data provided to the data-driven logic) which can otherwise not be known or may be difficult to implement as an analytical logic form.

The data-driven logic may be in software and/or hardware form, for example, executable via one or more computing units.

It shall be appreciated that in the present context, the data-driven logic refers to a trained mathematical logic which is parametrized according to the respective training data set. For example, the anatomy data-driven logic is parameterized via its respective training data to detect one or more anatomical features. An untrained logic lacks this information. Hence, the untrained logic or model is incapable for performing a desired detection. Feature engineering and training with the respective training datasets thus enables parametrization of the untrained logic. The result of such a training phase is the respective data-driven model, which as a result of the training process, preferably solely as a result of the training process, provides interrelations and logical capability related to the purpose for which the respective data-driven logic is to be used.

The data-driven logic preferably comprises, or it is a regression logic. In some cases, the data-driven logic may be combined with, or it may further include an analytical logic. The analytical logic may describe the relation between its input and output as a function or one or more mathematical equations or logical criteria. Thus, any of the data-driven logics described herein may even be a hybrid logic. A hybrid logic refers to a logic which comprises first-principles parts, so called white-box described via a set of equations e.g., mathematical equations, as well as data-driven parts as explained previously. The data-driven part may also be called black-box logic or model. Hence, the data-driven logic in some cases may be a combination of white-box logic or analytical logic, and black-box logic. In some cases, the data-driven logic may be, or it may even have a grey-box part. A grey-box logic in this context refers to a logic or model which combines a partial analytical structure with a data-driven part using training data to complete the model.

"Anatomy data-driven logic" may refer to a data-driven logic which is trained or pretrained in a supervised manner using anatomy training data comprising historical digital impressions and/or cell arrangements with annotation data specifying respective anatomical features. Alternatively, or additionally, the anatomy data-driven logic may be trained in an unsupervised manner. Hence, the anatomy data-driven logic is a logic which has already undergone a training phase and is ready to be deployed in an inference mode.

When in inference mode, the anatomy data-driven logic may be provided with the digital impression as input. The anatomy data-driven logic provides the anatomy data as an output. The anatomy data comprise at least one annotation related to at least one anatomical feature detected in the digital impression.

According to an aspect, the anatomy data-driven logic performs segmentation operation on the cells for detecting the anatomical features. Alternatively, or additionally, the anatomy data-driven logic performs detection and/or localization operations for detecting the anatomical features.

Thus, in general, the anatomy data-driven logic extracts one or more features of interest, or the anatomical features, by analyzing the digital impression.

Thus, by doing so, semantic understanding of the digital impression or cells, may be obtained.

"Segmentation operation" in the present context refers to a computerized processing operation which concludes with demarcation of at least one anatomical feature e.g., with a computer-generated feature boundary. The boundary may for example be realized by grouping the cells into a plurality of groups, at least one of the groups be in associated with a unique anatomical feature.

As it was discussed, the anatomy data-driven logic may perform the segmentation operation for detecting the one or more pre-selected anatomical features. Thus, the anatomy data-driven logic may segment the cells for detection. Thus, the anatomy data-driven logic may extract attributes or one or more objects or features of interest by analyzing the digital impression, or more specifically, the cells. The anatomy data-driven logic may classify every cell in the digital impression to a class according to its context such that each cell is assigned to a specific object. A non-limiting example of a segmentation operation when applied using the present teachings may be an outline around a feature such as a pre-selected anatomical feature, such as a specific tooth or even a group of teeth. Another non-limiting example is an outline around a specific pathology. Said segmented outline corresponds to the shape of the respective anatomical feature present in the digital impression.

There may be a plurality of segmented objects in the digital impression.

In addition to the data-driven approach, the segmentation operation may be supplemented by an analytical model using any suitable segmentation algorithm, e.g., point and line detection, edge linking, and/or thresholding method, histogram, adaptive, and their likes.

The segmentation operation may be based on classical image, 3D segmentation. Thus, objects or regions of interest may include tooth and/or teeth surface, gingiva surface, foreign objects, brackets, scan body surfaces, but also point- or line-based features such as tooth cusps and fissures. The features of interest may not be limited to cell surface or mesh surface, such as e.g., teeth centers of gravity, but alternatively, or additionally, the features may also be subdivided into sub-features such as buccal vs. lingual tooth surface. Based on the segmentation, other metrics such as tooth numbers may be derived.

The output of the segmentation operation may be used for analyzing the cells with respect to the cell criteria. For example, a semantic output of the segmentation operation may be used to evaluate presence or absence of a certain anatomical feature in the digital impression. Alternatively, or additionally, the segmentation operation output may comprise cell related quality, e.g., any one or more of, consistency, watertightness, flipped triangles or normals, normal sets, triangle or polygon size, or self-intersections.

"Cell analyzer data-driven logic" refers to a data-driven logic which is used at least partially for performing the cell analysis, e.g., analyzing the cells with respect to the cell criteria. Additionally, in some cases, the cell analyzer data-driven logic may also determine which of the dental procedures the digital impression is usable for and/or which of the dental procedures the digital impression is not usable for.

The cell analyzer data-driven logic refers to a data-driven logic which is trained with cell analyzer training data comprising a plurality of historical digital impressions each of which are linked to at least one dental procedure which the respective digital impression is suitable for and/or at least one dental procedure which the respective digital impression is unsuitable for. Those skilled in the art shall appreciate that the cell criteria may be inherently defined by the cell analyzer data-driven logic by virtue of being trained from the cell analyzer training data. In some cases, an additional analytical logic may be provided to the cell analyzer data-driven logic for additional cell criteria. The additional analytical logic may be placed in series to the data-driven part, upstream and/or downstream, and/or it may be arranged in a parallel manner with respect to the data-driven part.

"Combined logic" refers to a logic comprising at least of the two different data-driven logics disclosed herein. The combined logic may be pretrained in a supervised manner using end-to-end training process. The end-to-end training process in this context refers to at least partially annotated data comprising inputs and corresponding outputs. In some cases, the combined logic may be trained fully or partially in an unsupervised manner.

"Initiation signal" refers to a signal generated in response to the cell analysis. The initiation signal may be used to initiate a dental procedure, manually and/or automatically. For example, the initiation signal may be provided at the HMI for informing the user to initiate the dental procedure. Alternatively, or additionally, the dental procedure may be initiated automatically.

Alternatively, or additionally, the initiation signal may be used to initiate a cell corrective procedure for making the digital impression suitable for at least one predetermined dental procedure. Similar to the above, the initiation signal may be provided at the HMI for informing the user to initiate the cell corrective procedure. Alternatively, or additionally, the cell corrective procedure may be initiated automatically. According to an aspect, the user is guided to perform the cell corrective procedure. For example, the user may be guided to the at least one location or part of the digital impression in which of the cells should be improved. The user may even be guided through the entire process, for example, by continuously or intermittently evaluating the digital impression whether it has become suitable for the predetermined digital impression.

"Termination signal" refers to another signal generated in response to the cell analysis, however in some cases the initiation signal and the termination signal may be the same signal.

The termination signal may be used, for example, for terminating the acquisition of the digital impression in response to the analysis, manually and/or automatically. For example, the termination signal may be provided at the HMI for informing the user to terminate the acquisition of the digital impression. Alternatively, or additionally, the acquisition of the digital impression may be terminated automatically. It shall be appreciated that the termination signal may also be used as the initiation signal for initiating the dental procedure in any of the manners explained above.

An advantage of such termination can be that no extra time needs to be spent on acquiring the digital impression further as soon as the digital impression meets the cell criteria for predetermined one or more of the dental procedures.

"Computing unit", "computing device", "processing unit" or "processing device" may comprise, or it may be, a processing means or computer processor such as a microprocessor, microcontroller, or the likes, having one or more computer processing cores.

That two or more components are "operatively" coupled or connected shall be clear to those skilled in the art. In a non-limiting manner, this means that there may be at least one communicative connection between the coupled or connected components e.g., they are the network interface or any suitable interface. The communicative connection may either be fixed, or it may be removable. Moreover, the communicative connection may either be unidirectional, or it may be bidirectional. Furthermore, the communicative connection may be wired and/or wireless. In some cases, the communicative connection may also be used for providing control signals.

"Computer processor" refers to an arbitrary logic circuitry configured for performing basic operations of a computer or system, and/or, generally, to a device which is configured for performing calculations or logic operations. In particular, the processing means or computer processor may be configured for processing basic instructions that drive the computer or system. As an example, the processing means or computer processor may comprise at least one arithmetic logic unit ("ALU"), at least one floating point unit ("FPU"), such as a math coprocessor or a numeric coprocessor, a plurality of registers, specifically registers configured for supplying operands to the ALU and storing results of operations, and a memory, such as an L1 and L2 cache memory. In particular, the processing means or computer processor may be a multi core processor. Specifically, the processing means or computer processor may be or may comprise a central processing unit ("CPU"). the processing means or computer processor may be a complex instruction set computing ("CISC") microprocessor, reduced instruction set computing microprocessor ("RISC"), Very long instruction word ("VLIW") microprocessor, a processor implementing other instruction sets or processors implementing a combination of instruction sets. The processing means may also be one or more special purpose processing devices such as an application specific integrated circuit ("ASIC"), a field programmable gate array ("FPGA"), a complex programmable logic device ("CPLD"), a digital signal processor ("DSP"), a network processor, or the like. The methods, systems and devices disclosed herein may be implemented as software in a DSP, in a microcontroller, or in any other side processor such as hardware unit within an ASIC, CPLD, or FPGA. It is to be understood that the term processing means or processor may also refer to one or more processing devices, such as a distributed system of processing devices located across multiple computer systems (such as cloud computing), and is not limited to a single device unless otherwise specified.

"Memory storage" may refer to a device for storage of information, in the form of data, in a suitable storage medium. Preferably, the memory storage is a digital storage suitable for storing the information in a digital form which is machine readable, for example digital data that are readable via a computer processor. The memory storage may thus be realized as a digital memory storage device that is readable by a computer processor. Further preferably, the memory storage on the digital memory storage device may also be manipulated by a computer processor. For example, any part of the data recorded on the digital memory storage device may be written and or erased and or overwritten, partially or wholly, with the new data by the computer processor.

"Connectivity interface" or "communication interface" refers to a software and/or hardware interface for establishing communication such as transfer or exchange of signals or data. The communication may either be wired, or it may be wireless. Connectivity interface is preferably based on, or it supports one or more communication protocols. The communication protocol may be a wireless protocol, for example: short distance communication protocol such as Bluetooth^{®}, or Wi-Fi, or long communication protocols such as cellular or mobile network, for example, second generation cellular network ("2G"), 3G, 4G, long term evolution ("LTE"), or 5G. Alternatively, or in addition, the connectivity interface may even be based on proprietary short distance or long-distance protocol. The connectivity interface may support any one or more standards and/or proprietary protocols.

"Network" discussed herein may be any suitable kind of data transmission medium, wired, wireless, or their combination. A specific kind of network is not limiting to the scope or generality of the present teachings. The network can hence refer to any suitable arbitrary interconnection between at least one communication end point to another communication end point. Network may comprise one or more distribution points, routers or other types of communication hardware. The interconnection of the network may be formed by means of physically hard wiring, optical and/or wireless radio frequency ("RF") methods. The network specifically may be, or it may comprise, a physical network fully or partially made by hard wiring, such as a fiber optical network or a network fully or partially made by electrically conductive cables or a combination thereof. The network may at least partially comprise the Internet.

"Network interface" refers to a device or a group of one or more hardware and/or software components that allow an operative connection with the network.

### BRIEF DESCRIPTION OF THE SEVERAL VIEWS OF THE DRAWINGS

Certain aspects of the present teachings will now be discussed with reference to the accompanying drawings that explain said aspects by the way of examples. Since the generality of the present teachings is not dependent on it, the drawings may not be to scale. Method and system aspects may be discussed in conjunction for ease of understanding. Certain features shown in the drawings may be logical features that are shown together with physical features for the sake of understanding and without affecting the generality or scope of the present teachings.
FIG. 1 illustrates a block diagram of a scanning system;
FIG. 2 illustrates a flowchart of an aspect of the present teachings.

### DETAILED DESCRIPTION

In accordance with example aspects described herein, methods, systems and computer readable storage media can be provided for improving digital dentistry.

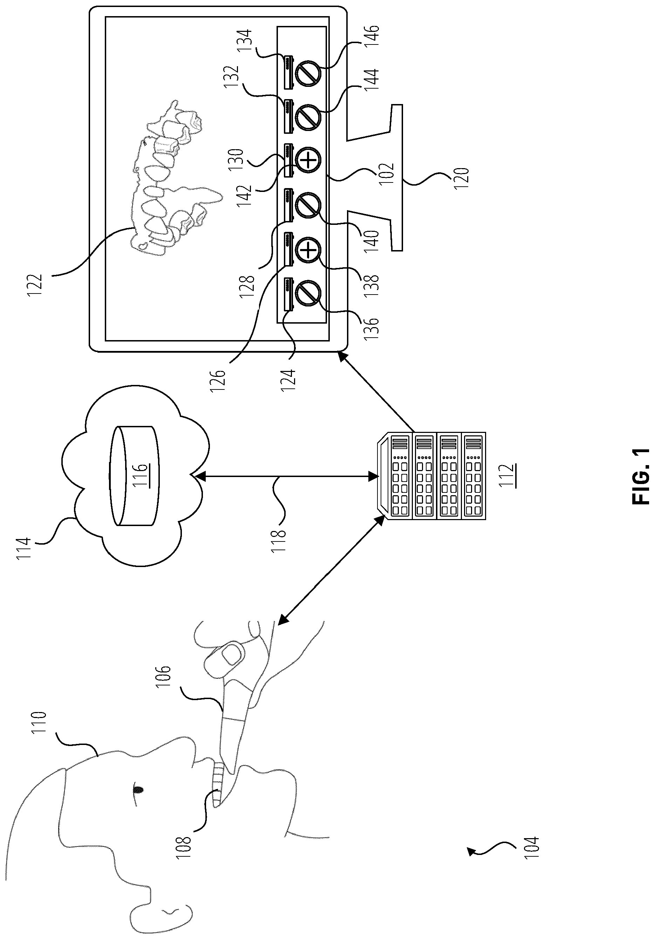

FIG. 1 shows a block diagram 104 of a digital dentistry system for recording craniofacial anatomy features. The system in this example is shown comprising a dental scanner 106. The dental scanner 106 is being used for intraoral scanning of dental condition or oral anatomy 108 of a patient 110. The dental scanner 106 is being used for providing a digital impression 122 of the oral anatomy of the patient 110. In this example, the digital impression 122 is shown presented on a human machine interface ("HMI") device 120. The digital impression 122 may be acquired as a part of a routine check, or for a specific purpose, for example, for performing one or more dental procedures and/or manufacturing one or more dental objects for the patient 110. The practitioner performing the scan may have to rely upon their experience to decide when the digital impression 122 is good enough. It may however be difficult for the practitioner to judge the quality of the digital impression 122 for different purposes. Sometimes, it might not be good enough, in which case the scan may have to be repeated partly or fully. In other times, the scan might be performed more intensively than required for the purpose.

The dental scanner 106 is operatively connected to a computing module 112, which comprises at least one computing unit. In some cases, the computing module 112 may be located at least partially within the dental scanner 106. In some cases, the computing module 112 may at least partially be a part of a cloud service 114. Thus, the computing module 112 may be a local device at the location of the dental scanner 106 and/or at least partially a remotely located device or system, e.g., one or more cloud services 114 and/or a remote server. The computing module 112 is also operatively connected to a memory storage or a database 116, which may at least partially be a part of the cloud service 114. In this case, the database 116 is shown provided at the cloud service 114. The dental scanner 106 may connect to an external computing module 112 via a connectivity interface (not explicitly shown in FIG. 1). Any local devices, e.g., the dental scanner 106 and/or the local computing module 112 may connect to the cloud service 114 via one or more networks 118. A network interface (not explicitly shown in FIG. 1) may be used to connect the local devices to the remote devices, e.g., the cloud service 114. In some cases, the network interface and the connectivity interface may be the same unit.

Optionally, the computing module 112 may be operatively connected to the HMI 120, which in FIG. 1 is shown as a computer screen. The connection between the computing module 112 and the HMI 120 may be via an output interface (not explicitly shown in FIG. 1), which may for example be a computer port or bus, e.g., display port, HDMI, USB, or their like. However, the output interface may even be an API for providing data to one or more computing units on the HMI 120 side. In some cases, the output interface may be a part of the network interface and/or the connectivity interface. Accordingly, it is possible that the three interfaces are the same device.

The dental scanner 106 comprises a sensor unit (not explicitly shown in FIG. 1) for imaging the surface of the oral anatomy 108 of the patient 110. A plurality of dental images of the oral anatomy 108 is generated via the dental scanner 106. Thus, during the scan, the dental scanner 106 is operated to capture the plurality of dental images of the oral anatomy 108. The dental images may be in the form of a data stream. These dental images are provided to the computing module 112. The computing module 112 is configured to provide a digital impression 122 from the dental images. The digital impression 122 is comprised of a plurality of cells, which dependent upon the kind of digital impression 122 may be point cloud, mesh, voxels, tetrahedrons, or their combination. The database 116 may be provided with data related to a plurality dental procedures and cell criteria corresponding to each of the dental procedures. Additionally, the digital impression 122 may also be provided at the database 116.

Pursuant to the present teachings, the computing module 112 is configured to analyze the cells with respect to the cell criteria. In response to this cell analysis, it is determined which of the dental procedures the digital impression 122 is usable for and/or which of the dental procedures the digital impression 122 is not usable for.

A response to the analysis is shown provided at the HMI 120, where a panel 102 is displayed with the plurality of dental procedures. In this example, six different dental procedures, namely a first dental procedure 124, a second dental procedure 126, a third dental procedure 128, a fourth dental procedure 130, a fifth dental procedure 132 and a sixth dental procedure 134 are shown with names and/or icons in the panel 102. More specifically, as a response to the cell analysis, it is determined that the digital impression 122 is suitable for the second dental procedure 126 and for the fourth dental procedure 130, while it is unsuitable for the first dental procedure 124, the third dental procedure 128, the fifth dental procedure 132 and the sixth dental procedure 134. The practitioner is thus enabled to decide whether to further improve the digital impression 122 or terminate the acquisition, thereby saving time and improving comfort for the patient 110. The determination may be done in the form of quality scores which in this example are in a simple binary form for convenience for the practitioner. More specifically, a second quality score 138 and a fourth quality score 142 are shown as a "plus" icon which may represent that the digital impression 122 meets the cell criteria respectively for the second dental procedure 126 and the fourth dental procedure 130. The quality scores of the rest of the dental procedures, namely, a first quality score 136, a third quality score 140, a fifth quality score 144 and a sixth quality score 146 are each shown as a "block" icon representing that the digital impression 122 is unsuitable for the associated dental procedures, i.e., for the first dental procedure 124, the third dental procedure 128, the fifth dental procedure 132 and the sixth dental procedure 134. The quality scores may even be represented in more discrete levels, e.g., in a traffic light form with "green" meaning a good quality, "yellow" average or acceptable, while "red" meaning an unsuitable quality for a specific dental procedure. Alternatively, or additionally, the quality scores may be shown in numerical values, continuous or discrete. For example, the digital impression 122 is 80% suited for a particular dental procedure, and/or 23% suited to another dental procedure.

Optionally, as a response to the analysis one or more a dental procedure and/or a cell corrective procedure may be initiated, for example, via an initiation signal. Similarly, the acquisition of the digital impression 122 may be automatically terminated if it meets the cell criteria of one or more predetermined dental procedures.

Thus, the practitioner can be assisted in taking informed decisions while reducing scan time to obtain the digital impression 122 which is suited for one or more purposes.

Those skilled in the art shall appreciate that the above can apply for any kind of digital impression 122, not only those obtainable via an intraoral scan.

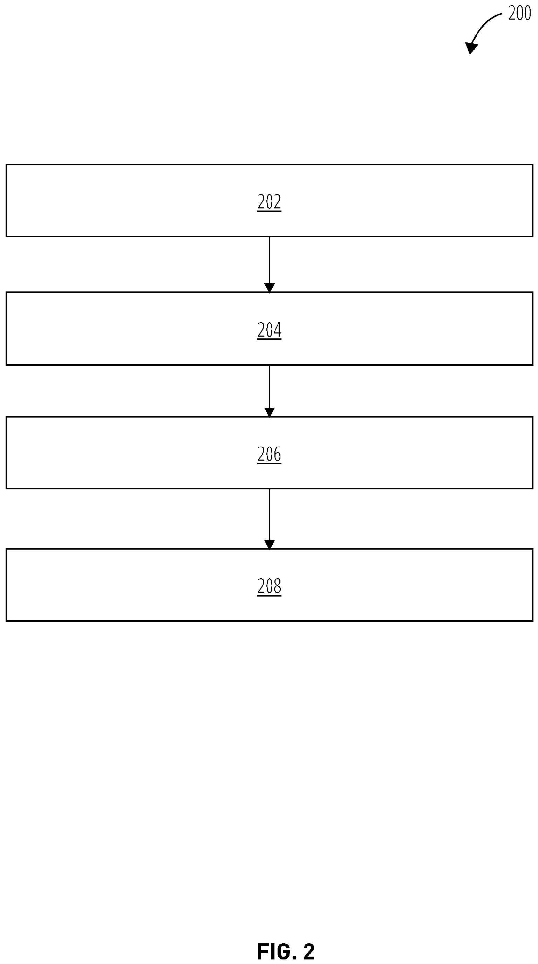

FIG. 2 shows a flowchart 200 exemplifying an aspect of the present teachings. The flowchart 200 can be implemented as a routine executable by one or more computing units, for example, operatively connected to a dentistry system or apparatus.

In block 202, it is provided a three-dimensional digital impression 122 of a craniofacial anatomy 108. The digital impression 122 comprises a plurality of cells, for example, mesh, point cloud, voxels or tetrahedrons.

In block 204, it is provided, a database 116 or at the database 116, a plurality of dental procedures 124, 126, 128, 130, 132 and 134, and their corresponding cell criteria.

In block 206, it is analyzed, the cells with respect to the cell criteria.

In block 208, it is determined in response to the analysis, which of the dental procedures the digital impression 122 is usable for and/or which of the dental procedures the digital impression 122 is not usable for.

The method steps may be performed in the order as shown listed in the examples or aspects. It should be noted, however, that under specific circumstances a different order may also be possible. Further, it is also possible to perform one or more of the method steps once or repeatedly. These steps may be repeated at regular or irregular time periods. Further, it is possible to perform two or more of the method steps simultaneously or in a timely overlapping fashion, specifically when some or more of the method steps are performed repeatedly. The method may comprise further steps which are not listed.

The word "comprising" does not exclude other elements or steps, and the indefinite article "a" or "an" does not exclude a plurality. A single processing means, processor or controller or other similar unit may fulfill the functions of several items recited in the claims. The mere fact that certain measures are recited in mutually different dependent claims does not indicate that a combination of these measures cannot be used to advantage. Any different signs in the claim should not be construed as limiting the scope.

Further, it should be noted that in the present disclosure, the terms "at least one", "one or more" or similar expressions indicating that a feature or element may be present once or more than once typically may have been used only once when introducing the respective feature or element. Thus, in some cases unless specifically stated otherwise, when referring to the respective feature or element, the expressions "at least one" or "one or more" may not have been repeated, notwithstanding the fact that the respective feature or element may be present once or more than once.

Further, the terms "preferably", "more preferably", "particularly", "more particularly", "specifically", "more specifically" or similar terms are used in conjunction with optional features, without restricting alternative possibilities. Thus, any features introduced by these terms are optional features and are not intended to restrict the scope of the claims in any way. The present teachings may, as those skilled in the art will recognize, be performed by using alternative features. Similarly, the features introduced by "according to an aspect" or similar expressions are intended to be optional features, without any restriction regarding alternatives to the present teachings, without any restrictions regarding the scope of the present teachings and without any restriction regarding the possibility of combining the features introduced in such way with other optional or non-optional features of the present teachings.

Unless otherwise defined, all technical and scientific terms used herein have the same meaning as commonly understood by one of ordinary skill in the art to which the present teachings belong.

Various examples have been disclosed above for a method, a system, a device, a use, software program, and a computing unit comprising the computer program code for carrying out the methods herein disclosed. For example, it has been disclosed a method for improving digital dentistry comprising: providing a 3D digital impression of a craniofacial anatomy; the digital impression comprising a plurality of cells, providing a database of a plurality of dental procedures and their corresponding cell criteria; analyzing the cells with respect to the cell criteria; determining, in response to the analysis, which of the dental procedures the digital impression is usable for and/or which of the dental procedures the digital impression is not usable for. The present teachings also relate to a system, data, software product and a storage medium. Those skilled in the art will understand however that changes and modifications may be made to those examples without departing from the spirit and scope of the accompanying claims and their equivalence. It will further be appreciated that aspects from the method and product embodiments discussed herein may be freely combined.

Any headings utilized within the description are for convenience only and have no legal or limiting effect.

## Claims

1. A computer-implemented method for improving digital dentistry, which method comprises:
- providing a three-dimensional digital impression of a craniofacial anatomy; wherein the digital impression comprises a plurality of cells,
- providing a database of a plurality of dental procedures and their corresponding cell criteria;
- analyzing the cells with respect to the cell criteria;
- determining, in response to the analysis, which of the dental procedures the digital impression is usable for and/or which of the dental procedures the digital impression is not usable for.

2. The method of claim 1, wherein the digital impression is in the form of surface data or volumetric data.

3. The method of claim 1 or 2, further comprising:
- detect, in response to the analysis, one or more deficiencies in the cells for usability in one or more of the dental procedures.

4. The method of any one of claims 1 to 3, further comprising:
- determining, in response to the analysis, at least one location or part of the digital impression in which the cells should be improved.

5. The method of any one of claims 1 to 4, wherein the analysis is performed while the digital impression is being acquired from a patient.

6. The method of any one of claims 1 to 5, further comprising:
- providing, at a human machine interface, one or more responses to the analysis.

7. The method of any one of claims 1 to 6, wherein the cell analysis is performed via a cell analyzer data-driven logic.

8. The method of any one of claims 1 to 7, further comprising:
- providing an initiation signal for initiating a dental procedure and/or a cell corrective procedure in response to the analysis.

9. The method of any one of claims 1 to 8, further comprising:
- determining, in response to the initiation signal, at least remedial action by which the cells should be improved, preferably for a pre-determined dental procedure.

10. The method of any one of claims 5 to 9, further comprising:
- generating a termination signal for terminating the acquisition of the digital impression in response to the analysis.

11. The method of any one of claims 1 to 10, further comprising:
- determining, in response to the analysis, at least one quality score for the cells.

12. The method of claim 11, further comprising:
- displaying, at the HMI, the at least one quality score, preferably a plurality of quality scores for different dental procedures.

13. A system comprising means for performing the steps of any of the above method claims.

14. A computer software product, or a non-transitory computer-readable storage medium storing the software, comprising instructions which when executed by a suitable one or more computing units cause any of the computing units to perform the steps of any of the above method claims.

15. A data storage medium storing a digital impression comprising the corrected cells as generated in any one of claims 8 to 12.

16. Use of a digital impression comprising the corrected cells as generated in any one of claims 8 to 12 for performing a dental procedure and/or for manufacturing a dental object.
